# EUROPEAN PATENT APPLICATION

(11) **EP 4 442 675 A1**
(43) Date of publication of application: **09.10.2024**
(21) Application number: 22901656.3
(22) Date of filing: 23.11.2022
(51) Int. Cl.: C07C 68/06, C07C 68/08, C07C 69/96

(54) **METHOD FOR PREPARING CARBONATE**

(30) Priority: 30.11.2021 KR 20210168077
(71) Applicant: LOTTE CHEMICAL CORPORATION, Seoul 05551 (KR)
(72) Inventor: HAN, Eunhye, Daejeon 34110 (KR); KIM, Wang Gyu, Daejeon 34110 (KR); BAEK, Mi Hwa, Daejeon 34110 (KR); CHOI, Jong Myung, Seoul 05551 (KR)
(74) Representative: Germain Maureau
(86) International application number: PCT/KR2022/018589
(87) International publication number: WO 2023/101298

(57) **Abstract**

The present invention relates to a method for producing a carbonate. The method for producing a carbonate according to the present invention is capable of removing a catalyst applied in the preparation process with high efficiency, without damaging an adsorbent.

## Description

### [Technical Field]

The present invention relates to a method for producing a carbonate. Specifically, the present invention relates to a method for efficiently removing a catalyst used in a carbonate production reaction.

### [Background Art]

As an organic solvent for a battery electrolyte, ethyl methyl carbonate (EMC) and diethyl carbonate (DEC) are mainly used. The EMC and DEC are prepared by a transesterification reaction of dimethyl carbonate (DMC) and ethanol (EtOH). The transesterification reaction is also a reversible reaction. In addition, the transesterification reaction may also be performed in the presence of a catalyst. At this time, a basic catalyst is mainly used as the catalyst. Reaction Formula 1 below shows the transesterification reaction (in which, MeOH is an abbreviation for methanol):

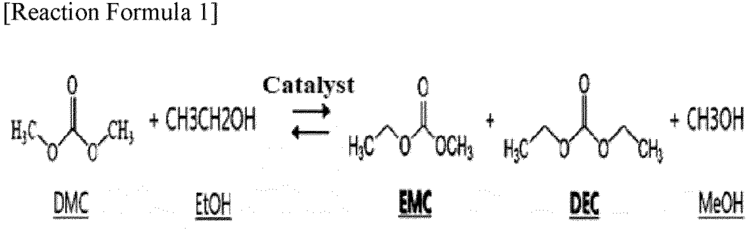

In the reaction, a basic catalyst (mainly a metal salt containing an alkali metal or alkaline earth metal as a metal component) is used as the catalyst. The catalyst has low solubility in carbonate, which is a reactant or product of the reaction. Therefore, if the catalyst is not separately removed, defects in subsequent processes may occur. This is because the precipitated catalyst causes column plugging or fouling in the subsequent process.

As a method of removing the catalyst, a method of filtering the catalyst through a filter has been considered. However, when the size of the catalyst is smaller than the pore size of the filter, or when alcohol contained in the product of the reaction dissolves the catalyst, the catalyst may not be filtered through the filter.

Patent Document 1 discloses a process of reacting DMC and alcohol in the presence of a basic catalyst (Sodium Methoxide, SME), producing EMC and DEC through a distilling process, and introducing a strainer (a device for separating solid materials) at the end of a reaction distillation column. In addition, Patent Document 2 discloses the content of removing a catalyst by installing a porous tray inside a reaction distillation tower. However, Patent Documents 1 and 2 still include a problem of plugging a distillation tower due to catalyst precipitation in a concentrate at the bottom of the distillation tower.

### [Prior Arts]

### [Patent Documents]

(Patent Document 1) CN 103804124 B
(Patent Document 2) KR 10-1668571 B.

### [Disclosure]

### [Technical Problem]

An object of the present invention is to remove a catalyst applied in a preparation process of a carbonate with high efficiency without damaging an adsorbent.

### [Technical Solution]

According to a first exemplary embodiment of the present invention, there is provided a method for producing a carbonate including: a reaction step of reacting raw materials containing dimethyl carbonate and ethanol in the presence of a catalyst to prepare a reaction product stream containing a catalyst, ethyl methyl carbonate, diethyl carbonate and methanol; an adsorption step of adding an acidic adsorbent into the reaction product stream to induce adsorption of the catalyst and the adsorbent; an adsorbent removal step of removing the adsorbent adsorbed with the catalyst to prepare a reaction product stream from which the adsorbent has been removed; and a product separation step of separating ethyl methyl carbonate or diethyl carbonate from the reaction product stream from which the adsorbent has been removed, in which the adsorbent is an aluminum-based adsorbent.

According to a second exemplary embodiment of the present invention, there is provided a method for producing a carbonate including: a reaction step of reacting raw materials containing dimethyl carbonate and ethanol in the presence of a catalyst to prepare a reaction product stream containing a catalyst, ethyl methyl carbonate, diethyl carbonate and methanol; an adsorption step of supplying the reaction product stream to an adsorption tower including an acidic adsorbent to induce adsorption of the catalyst and the adsorbent; and a product separation step of separating ethyl methyl carbonate or diethyl carbonate from the reaction product stream passing through the adsorption tower, in which the adsorbent is a bead-shaped aluminum-based adsorbent.

### [Advantageous Effects]

According to the present invention, the method for producing the carbonate is capable of removing a catalyst applied in the preparation process with high efficiency, without damaging an adsorbent.

### [Description of Drawings]

FIG. 1 is a schematic diagram of a method for producing a carbonate according to a first exemplary embodiment of the present invention.
FIG. 2 is a schematic diagram of a method for producing a carbonate according to a second exemplary embodiment of the present invention.
FIG. 3 is a photograph when an adsorbent is added in Comparative Example 7 (left) and Example 3 (right) of the present invention.

### [Best Mode of the Invention]

Hereinafter, the contents of the present invention will be described in more detail.

As described above, the present invention is intended to efficiently recover or remove a catalyst used in a reaction of producing carbonate, specifically ethyl methyl carbonate and diethyl carbonate. As a result, the product may be obtained with high purity. A carbonate compound produced above may be usefully applied as a solvent for a secondary battery electrolyte.

As described above, in the present invention, the product of the reaction may not dissolve the catalyst in the reaction. If the process is performed after the reaction without removing the catalyst, the catalyst will be precipitated, clogging pipes, etc., and cause column plugging or fouling, which is a cause of process defects. The catalyst may also be filtered using a filter, but the catalyst may be dissolved in alcohol, which is a by-product of the reaction, and the dissolved catalyst may pass through the filter as it is. Since a product stream containing the dissolved catalyst also causes postprocess defects, it is necessary to remove even the dissolved catalyst.

In the present invention, the catalyst is removed with high efficiency by using an adsorbent or adsorption tower. In addition, it is possible to remove the catalyst with high efficiency without damaging the adsorbent by applying the method of the present invention. Therefore, it is possible not only to prepare a carbonate with high purity, but also to secure economic efficiency of a process by applying the method of the present invention.

Hereinafter, the method using the adsorbent itself is referred to as a first exemplary embodiment of the present invention, and the method using the adsorption tower is referred to as a second exemplary embodiment of the present invention.

### First Exemplary Embodiment

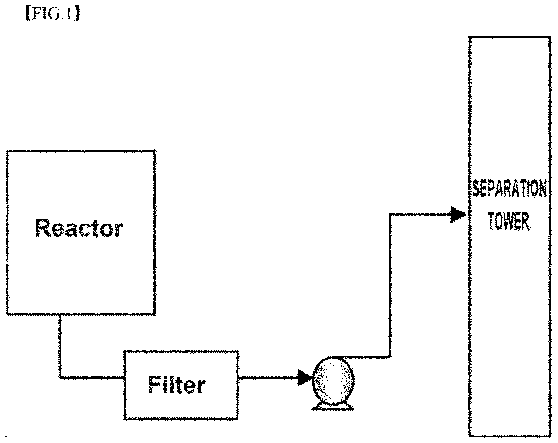

A method for producing a carbonate according to a first exemplary embodiment of the present invention includes at least four steps. The method according to the first exemplary embodiment of the present invention includes using the adsorbent itself as a main content. FIG. 1 is a schematic diagram of a process of performing the method according to the first exemplary embodiment of the present invention.

The method for producing the carbonate according to the first exemplary embodiment of the present invention includes at least a reaction step, an adsorption step, an adsorbent removal step, and a product separation step. Hereinafter, each step will be described in more detail.

In the method for producing the carbonate according to the first exemplary embodiment of the present invention, in the reaction step, a reaction product stream including a catalyst, ethyl methyl carbonate, diethyl carbonate and methanol is prepared by reacting raw materials containing dimethyl carbonate and ethanol in the presence of a catalyst.

Dimethyl carbonate (DMC) and ethanol (EtOH) produces ethyl methyl carbonate (EMC) or diethyl carbonate (DEC) as a main product and methanol (MeOH) as a by-product through a transesterification reaction.

The conversion rate of reactions, selectivity and yield of target products may vary depending on the composition of raw materials. Therefore, the composition between the reactants in the raw materials may be adjusted in consideration of maximizing a preferable target product, as well as the conversion rate.

In an exemplary embodiment, the raw materials may include dimethyl carbonate (DMC) and ethanol (EtOH) at a weight ratio (EtOH/DMC) within the range of 0.1 to 10.

In an exemplary embodiment, an appropriate reactor for performing the reaction of the raw materials may be selected. In the present invention, the catalyst applied to the reaction for producing the carbonate is intended to be removed from the reaction product stream. Therefore, the reactor applied in the present invention is a reactor that leaves at least the catalyst in the reaction product stream.

Meanwhile, a fixed bed reactor charges and fixes the catalyst in the reactor. In other words, when a catalytic reaction proceeds in the fixed bed reactor, there is almost no catalyst in the reaction product. Therefore, the reactor mentioned in the present invention is at least not the fixed bed reactor. That is, the reactor mentioned in the present invention is a reactor containing at least a catalyst flowing in the reactor. Such a reactor is also called fluidized bed reactor in the art.

In the present invention, a batch reactor or a continuous flow reactor may be used as the reactor. Specifically, in the present invention, the effect of the present invention may be verified using the batch reactor. In addition, when the method of the present invention is applied to actual product production, the continuous flow reactor may be applied.

In the present disclosure, the batch reactor is a reactor in which supply of reactants and discharge of products occur once.

In the present disclosure, the continuous flow reactor is literally a reactor in which the supply of reactants and the discharge of products occur continuously.

The continuous flow reactor may be broadly classified into two types. The first type is a continuous stirred tank reactor or mixed flow reactor (CSTR or MFR), and the second type is a plug flow reactor (PFR). Among them, the CSTR may maintain constant composition of components in the reactor. In the present invention, a uniform reaction may be performed by applying the CSTR as the reactor. That is, in an exemplary embodiment of the present invention, the reaction step may be performed in the continuous stirred tank reactor (CSTR).

Matters related to the size of the reactor are not particularly limited in the present invention. In the present invention, the size of the reactor may be determined by comprehensively considering the speed of the reaction, the yield and/or selectivity of a target product, the conversion rate of the reactant, etc.

The chemical reaction used in the present invention is a transesterification reaction of DMC and EtOH. In the chemical reaction, the conversion rate of the reactant, the selectivity of the target product, etc. in the reaction vary depending on the composition of a reactant, the content of a catalyst, a reaction temperature, etc. Here, the catalyst content and the reaction temperature affect the reaction rate.

Meanwhile, in the reaction applied in the present invention, the product composition mentioned above varies greatly depending on the composition of the reactants in the raw materials, as described above. In addition, when the reaction is performed in a specific reactor, such as a CSTR, the product composition may be particularly affected by the reactant composition. In this case, the catalyst content, the temperature, etc. may not have a significant effect.

In addition, since the reaction may not proceed 100%, the product stream may also include a certain amount of DMC and EtOH contained in the raw materials.

In the method of the present invention, the reaction of the raw materials proceeds in the presence of the catalyst. The catalyst applicable to the reaction may be a basic catalyst. The basic catalyst may include sodium methoxide (NaOCH₃), sodium ethoxide (NaOC₂H₅), potassium hydroxide (KOH), sodium hydroxide (NaOH), or a combination thereof. Here, NaOCH₃ may be typically used as the catalyst. The carbonates (DMC, EMC or DEC) in the reactants or reaction products do not dissolve the catalyst, but alcohols (ethanol or methanol) in the reactants or reaction products may dissolve the catalyst. Accordingly, the product stream may contain a certain amount of sodium cations (Na⁺). The sodium cations may not be filtered using a general filter, and may be precipitated in a purification process, causing process defects.

The present invention is specifically intended to solve problems that occur depending on the application of such a catalyst.

In the method for producing the carbonate according to the first exemplary embodiment of the present invention, in the adsorption step, the adsorption of the catalyst and the adsorbent is induced by adding an acidic adsorbent to the reaction product stream. Specifically, in the adsorption step, the adsorbent may adsorb the catalyst through an ion exchange reaction between metal cations of the catalyst and hydrogen ions of the adsorbent. Here, the acidic adsorbent may refer to a metal salt and the like that release hydrogen ions or other metal cations when the adsorbent is dissociated in water.

In addition, the adsorbent is an aluminum-based adsorbent. The aluminum-based adsorbent means an adsorbent containing at least aluminum in a molecular structure. For example, the adsorbent may be aluminum silicate (Si-Al) or boehmite (γ-AlO(OH)). Meanwhile, a silicon-based adsorbent such as silica gel may not achieve desired catalyst removal efficiency in the present invention.

For example, when using an ionic salt with sodium as a metal ion as the catalyst and using boehmite as an adsorbent, the adsorbent may adsorb catalyst residues (Na metal or Na ions) through the following mechanism.

*-AlO*⁻]···*H*⁺ + *Na*⁺ ↔ *-AlO*⁻]···*Na*⁺ + *H*⁺

In the reaction formula, the O- site may remove not only sodium but also other basic and corrosive metals (e.g., Li, Be, Mg, K, Ca, Rb, Sr, Cs, Ba, etc.) remaining in the product stream.

In addition, when a basic catalyst such as NaOCH₃ is used as the catalyst, hydrogen ions released in the process may be subjected to an ion exchange reaction with the catalyst remaining in the reaction product to form methanol. Then, the methanol may also be separated from the product stream, recycled, and then added to the reaction process along with the raw materials. This is because the above-described basic catalyst may also be supplied in the form of a solution dissolved in methanol. The process of producing methanol is as follows.

*-OCH*3*⁻* ···*Na*⁺ + *H*⁺ ↔ *CH*3*OH* + *Na*⁺

The amount of adsorbent added to the product stream may also be appropriately adjusted. For example, in the present invention, in the adsorption step, 0.5 wt% to 20 wt% of the adsorbent based on the raw materials may be added. In another exemplary embodiment, the content of the adsorbent may be in the range of 1 wt% to 10 wt%, or in the range of 1 wt% to 5 wt%.

In the method for producing the carbonate according to the first exemplary embodiment of the present invention, in the adsorbent removal step, the adsorbent adsorbed with the catalyst is removed to produce a reaction product stream from which the adsorbent has been removed. This is because the adsorbent adsorbed with the catalyst is present as a solid in the reaction product stream, which may cause defects in the subsequent process. This is also a difference from the method according to a second exemplary embodiment of the present invention to be described below. That is, before purifying the product stream containing the adsorbent adsorbed with the catalyst in the present invention, the adsorbent adsorbed with the catalyst needs to be removed from the product stream.

In an exemplary embodiment, in the adsorbent removal step, the adsorbent adsorbed with the catalyst may be removed through filtering. If the reaction product properly passes through a filter having an appropriate pore size, the adsorbent will be adsorbed with the catalyst and present in a solid state. Accordingly, the catalyst and the adsorbent added for removal of the catalyst may be removed from the product stream.

In the method for producing the carbonate according to the first exemplary embodiment of the present invention, in the product separation step, ethyl methyl carbonate or diethyl carbonate is separated from the reaction product stream from which the adsorbent has been removed.

Among the products of the chemical reaction used in the present invention, a target product is a carbonate compound. Currently, DEC is used as a solvent for a secondary battery electrolyte, but as mentioned above, there is also a significant demand for EMC. Therefore, in the present invention, depending on business importance, a process of separating a specific compound (DEC or EMC) from the carbonates including in the product stream as the target product may be performed.

In an exemplary embodiment, in the product separation step, diethyl carbonate or ethyl methyl carbonate may be separated by using a flash drum from the reaction product stream from which the adsorbent is removed. This is because the catalyst and the adsorbent have been significantly removed from the product stream. That is, in the present invention, such a separation process may be performed without an additional purification process. In other words, in the present invention, the separation process may be performed with simple equipment (e.g., flash drum), and thus separate purification equipment is not required.

The method according to the first exemplary embodiment of the present invention may further include a preliminary catalyst removal step of filtering the reaction product stream, after the reaction step and before the adsorption step. That is, a significant amount of catalyst may be removed from the reaction product by using a filter or the like before removing the catalyst included in the reaction product with the adsorbent. In the present invention, the step is mainly used to solidify catalyst cations in a dissociated state from the adsorbent. Therefore, when the catalyst that is already in a solid state is removed in advance, it is possible to further improve the catalyst removal efficiency by applying the adsorbent.

In addition to the steps, the method according to the first exemplary embodiment of the present invention may include all other steps known to be necessary in the process for producing the carbonate and the process used for removing the catalyst used therein.

Next, a second exemplary embodiment of the present invention will be described.

### Second Exemplary Embodiment

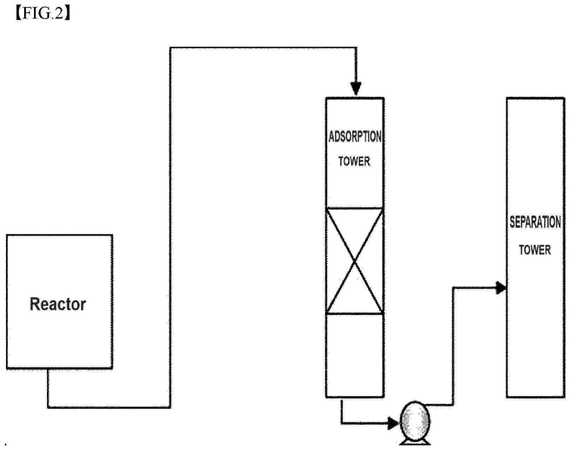

A second exemplary embodiment of the present invention includes at least three steps. The method according to the second exemplary embodiment of the present invention includes using an adsorption tower as a main content. FIG. 2 is a schematic diagram of a process of performing the method according to the second exemplary embodiment of the present invention. The second exemplary embodiment of the present invention includes at least a reaction step, an adsorption step, and a product separation step.

In the method for producing the carbonate according to the second exemplary embodiment of the present invention, in the reaction step, a reaction product stream including a catalyst, ethyl methyl carbonate, diethyl carbonate and methanol is prepared by reacting raw materials containing dimethyl carbonate and ethanol in the presence of a catalyst.

The corresponding step is performed in the same manner as the contents mentioned in the first exemplary embodiment described above. Therefore, the contents about the reaction step of the first exemplary embodiment may also be applied to the reaction step of the second exemplary embodiment.

In the second exemplary embodiment, the raw materials may include dimethyl carbonate (DMC) and ethanol (EtOH) at a weight ratio (EtOH/DMC) within the range of 0.1 to 10.

Accordingly, even in the second exemplary embodiment of the present invention, a continuous flow reactor may be used as the reactor in which the reaction occurs. In addition, matters related to the size of the reactor are not particularly limited in the present invention. In the present invention, the size of the reactor may be determined comprehensively by considering the speed of the reaction, the yield and/or selectivity of a target product, the conversion rate of the reactants, etc.

Even in the second exemplary embodiment of the present invention, like the first exemplary embodiment, the reaction is performed in the presence of the catalyst. Accordingly, matters related to the catalyst may also be applied with the contents mentioned in the first exemplary embodiment as they are. In addition, like the first exemplary embodiment, the second exemplary embodiment of the present invention is intended to solve problems which may occur when abovementioned catalysts are applied.

In the method for producing the carbonate according to the second exemplary embodiment of the present invention, adsorption of the catalyst takes place when the reaction product stream is supplied to the adsorption tower containing an acidic adsorbent. The corresponding content is different from the first exemplary embodiment.

First, regarding the catalyst itself used in the second exemplary embodiment, the catalyst mentioned in the first exemplary embodiment may be applied. That is, in the adsorption step of the second exemplary embodiment, the reaction product stream may be brought into contact with the acidic adsorbent for the adsorption of the catalyst and the adsorbent.

Specifically, in the adsorption step, the adsorbent may adsorb the catalyst through an ion exchange reaction between metal cations of the catalyst and hydrogen ions of the adsorbent. Here, the acidic adsorbents may refer to a metal salt and the like that release hydrogen ions or other metal cations when the adsorbent is dissociated in water. Therefore, the second exemplary embodiment may also be applied with the above-described adsorption mechanism of the adsorbent as it is.

In addition, the adsorbent is an aluminum-based adsorbent. The aluminum-based adsorbent means an adsorbent containing at least aluminum in a molecular structure. For example, the adsorbent may be aluminum silicate (Si-Al) or boehmite (γ-AlO(OH).

In addition, the adsorbent used in the second exemplary embodiment needs to be an aluminum-based adsorbent with a specific shape. Specifically, in the second exemplary embodiment of the present invention, the adsorbent applied in the adsorption step is a bead-shaped aluminum-based adsorbent. When an adsorbent is applied, for example, in the form of extrude or powder rather than in the form of the beads, even if the adsorbent is aluminum-based and acidic as the component, the catalyst removal efficiency desired in the present invention may not be achieved.

Unlike in the first exemplary embodiment, in the second exemplary embodiment, the product stream is supplied to the adsorption tower containing the adsorbent for the adsorption of the catalyst and the adsorbent. Specifically, the adsorption tower is filled with the adsorbent. More specifically, the adsorbent has a fixed state in the adsorption tower. Therefore, when the product stream passes through the adsorption tower, the catalyst is left in the adsorption tower as a result of adsorption of the catalyst and the adsorbent, and the product stream excluding the catalyst (or including a small amount of catalyst) flows out of the adsorption tower.

The amount of adsorbent filled in the adsorption tower may also be appropriately adjusted. In an exemplary embodiment, in the adsorption step, the reaction product stream may be supplied to the adsorption tower containing the adsorbent in the content range of 0.5 parts by weight to 100 parts by weight based on 100 parts by weight of the raw materials.

The method for producing the carbonate according to the second exemplary embodiment of the present invention does not include the adsorbent removal step, in which the adsorbent adsorbed with the catalyst is removed to produce a reaction product stream from which the adsorbent has been removed. The adsorbent adsorbed with the catalyst is present in a solid in the reaction product stream, which may cause defects in the subsequent process. However, in the exemplary embodiment, since the adsorbent is fixed in the adsorption tower, this problem is unlikely to occur. This is also a difference from the method according to the first exemplary embodiment of the present invention described above.

The method for producing the carbonate according to the second exemplary embodiment of the present invention includes the product separation step, in which ethyl methyl carbonate or diethyl carbonate is separated from the reaction product stream from which the adsorbent has been removed.

The corresponding step is performed in the same manner as the contents mentioned in the first exemplary embodiment described above. Therefore, the contents about the product separation step of the first exemplary embodiment may also be applied to the product separation step of the second exemplary embodiment.

Accordingly, in the second exemplary embodiment, in the product separation step, ethyl methyl carbonate or diethyl carbonate may be separated by using a flash drum from the reaction product stream from which the adsorbent is removed. This is because the catalyst and the adsorbent have been significantly removed from the product stream. That is, in the present invention, such a separation process may be performed without an additional purification process. In other words, in the present invention, the separation process may be performed with simple equipment (e.g., flash drum), and thus separate purification equipment is not required.

According to the second exemplary embodiment of the present invention, there is no need for a process of removing the adsorbent separately after removing the catalyst by adsorption from the product stream. In the second exemplary embodiment, the product stream from which the catalyst has been removed may be added by the separation process without any additional treatment. Therefore, in the second exemplary embodiment, the product separation step may be performed immediately after the adsorption step.

In addition to the steps, the method according to the second exemplary embodiment of the present invention may include all other steps known to be necessary in the process for producing the carbonate and the process used for removing the catalyst used therein.

Hereinafter, the contents of the present invention will be described in more detail with reference to Examples. However, the scope of the present invention is not limited to the following Examples.

### [Experimental Example 1] Gas chromatography

For qualitative and quantitative analysis of a reaction product, gas chromatography (GC) was performed on a sample in which 1 g of the product was mixed with 0.1 g of m-xylene. An YL6500GC product from YOUNGIN Chromas was used as the GC equipment, in which a GC column was DB-1 30 m * 0.32 mm from Agilent and a GC detector was FID. The reaction conversion rate of DMC, a preparation raw material, was calculated as mole% of consumption to supply, and the reaction selectivity of EMC, a target product, was calculated as mole% of EMC content to the total content of EMC and DEC produced, respectively.

### [Experimental Example 2] ICP-AES

The Na content was measured using inductively coupled plasma atomic emission spectroscopy (ICP-AES). An Optima 8300 model from Perkin Elmer was used. 10 g of an effluent sample was mixed with 10 ml of 70% nitric acid and 10 ml of water, heated at 250°C for 2 hours, and then analyzed.

### [Example 1]

### Reaction step

A solution was prepared by mixing 90.1 g of DMC and 46.07 g of EtOH. The solution was added to a CSTR reactor with a volume of 500 ml along with 0.09 g of basic catalyst SME (0.1% by weight compared to DMC). EMC and DEC were synthesized through a transesterification reaction while maintaining a reaction temperature in the reactor at 50°C. After allowing 1 hour of reaction time, the product stream was analyzed by GC. The results of 54% DMC conversion rate, 82% EMC selectivity, and 18% DEC selectivity were obtained. ICP-AES analysis was performed to determine the Na content in the reaction product. The Na content in the reaction product was 247.6 mg/L.

### Preliminary catalyst removal step

The reaction product was filtered at room temperature and reduced pressure using filter paper with a pore size of 1 µm. The Na content in the filtrate was 70.6 mg/L.

### Adsorption step

Subsequently, an additional adsorption process was performed to remove Na⁺. 5 g of a commercially available adsorbent (Powder type Aluminum Silicate, Kyowaad) was added to 100 g of the filtered reaction product (containing Na⁺) and then stirred at room temperature for 30 minutes.

### Adsorbent removal step

The adsorbent was removed using a PTFE syringe filter with a pore size of 1 µm. Thereafter, through ICP-AES analysis, it was confirmed that the Na content in the filtration solution was 0.45 mg/L.

### [Example 2]

A catalyst was removed in the same manner as in Example 1, except that a commercially available adsorbent (Powder type Boehmite, Osang Jaiel Co., Ltd.) was applied as an adsorbent. Thereafter, through ICP-AES analysis, it was confirmed that the Na content in the final filtration solution was 1.8 mg/L.

### [Comparative Example 1]

A catalyst was removed in the same manner as in Example 1, except that a commercially available adsorbent (Powder type Silica gel, Merck Co., Ltd.) was applied as an adsorbent. Thereafter, through ICP-AES analysis, it was confirmed that the Na content in the final filtration solution was 4.1 mg/L.

### [Comparative Example 2]

The same method as Example 1 was performed, but no additional processes were performed. As a result, through ICP-AES analysis, it was confirmed that the Na content in the final solution was 247.6 mg/L.

### [Comparative Example 3]

The same method as Example 1 was performed, but the adsorption step and the adsorbent removal step were not performed. As a result, through ICP-AES analysis, it was confirmed that the Na content in the final solution was 70.6 mg/L.

### [Comparative Example 4]

The same method as Example 1 was performed, but the adsorption step and the adsorbent removal step were not performed, and in the preliminary catalyst removal step, the catalyst was removed using a syringe filter made of PTFE with a pore size of 0.45 µm. As a result, through ICP-AES analysis, it was confirmed that the Na content in the final solution was 65.0 mg/L.

### [Example 3]

### Reaction step

A solution was prepared by mixing 90.1 g of DMC and 46.07 g of EtOH. The solution was added to a reactor with a volume of 500 ml along with 0.045 g of basic catalyst SME (0.05% by weight compared to DMC). EMC and DEC were synthesized through a transesterification reaction while maintaining a reaction temperature in the reactor at 50°C. After allowing 1 hour of reaction time, the product stream was analyzed by GC. The results of 54% DMC conversion rate, 82% EMC selectivity, and 18% DEC selectivity were obtained. ICP-AES analysis was performed to determine the Na content in the reaction product. The Na content in the reaction product was 130.3 mg/L.

### Adsorption step

An experiment was performed using 300 g of the reaction product as a raw material. Boehmite beads were prepared by calcining with an adsorbent at 300°C for 8 hours to remove an organic binder. A quartz tube was filled with 12 g of the adsorbent. Under the conditions of a raw material flow rate of 600 g/hr, a weight hour space velocity (WHSV) of 50 hr⁻¹, and room temperature, the raw materials were circulated in the quartz tube for 6 hours. Thereafter, it was confirmed through ICP-AES analysis that the Na content in the filtrate that passed through the adsorption tower (quartz tube) was 14.1 mg/L.

### [Example 4]

A catalyst was removed in the same manner as in Example 3, except for using Si-Al beads from which an organic binder was removed by calcining with an adsorbent at 300°C for 8 hours. It was confirmed through ICP-AES analysis that the Na content in the filtrate that passed through the adsorption tower (quartz tube) was 20.7 mg/L.

### [Comparative Example 5]

A catalyst was removed in the same manner as in Example 3, except for using a Boehmite extrudate from which an organic binder was removed by calcining with an adsorbent at 300°C for 8 hours. It was confirmed through ICP-AES analysis that the Na content in the filtrate that passed through the adsorption tower (quartz tube) was 2.41 mg/L.

### [Comparative Example 6]

### Preliminary catalyst removal step

The reaction product in the reaction step of Example 3 was filtered under conditions of room temperature and reduced pressure using filter paper with a pore size of 1 µm. ICP-AES analysis was performed, and the Na content in the filtrate was 77.2 mg/L. 300 g of the corresponding reaction product was applied to the adsorption step.

### Adsorption step

Silica gel W127 was prepared as an adsorbent. A quartz tube was filled with 12 g of the adsorbent. Under the conditions of a raw material flow rate of 600 g/hr, a weight hour space velocity (WHSV) of 50 hr⁻¹, and room temperature, the raw materials were circulated in the quartz tube for 6 hours. Thereafter, it was confirmed through ICP-AES analysis that the Na content in the filtrate that passed through the adsorption tower (quartz tube) was 44.7 mg/L.

### [Comparative Example 7]

A catalyst was removed in the same manner as Comparative Example 6, except for using Silica gel B 127 and Bead from which moisture was completely removed through pretreatment of an adsorbent at 180°C for 15 hours in Comparative Example 6. It was confirmed through ICP-AES analysis that the Na content in the filtrate that passed through the adsorption tower (quartz tube) was 29.4 mg/L.

### [Comparative Example 8]

A catalyst was removed in the same manner as in Comparative Example 7, but a filtration process using filter paper with a pore size of 1 µm was not performed. It was confirmed through ICP-AES analysis that the Na content in the filtrate that passed through the adsorption tower (quartz tube) was 68.7 mg/L.

The results of Examples 1 to 4 and Comparative Examples 1 to 8 were summarized in Tables 1 and 2 below.

**[Table 1]**

| Classification | | Ex. 1 | Ex. 2 | Com. Ex. 1 | Com. Ex. 2 | Com. Ex. 3 | Com. Ex. 4 |
|---|---|---|---|---|---|---|---|
| Na (mg/L) | Raw material | 247.6 | 247.6 | 247.6 | 247.6 | 247.6 | 247.6 |
| | 1 µm | 70.6 | 70.6 | 70.6 | - | 70.6 | - |
| | 0.45 µm | - | - | - | - | - | 65.0 |
| Adsorbent | | A | B | C | Absence | Absence | Absence |
| Adsorbent added amount (g/100 g compared to raw material) | | 5 | 5 | 5 | - | - | - |
| Na content (mg/L) | After passing through adsorbent | 0.45 | 1.8 | 4.1 | - | - | - |
| A: Si-Al powder | | | | | | | |
| B: Boehmite(Al) powder | | | | | | | |
| C: Silica gel powder | | | | | | | |

Examples 1 and 2 and Comparative Examples 1 to 4 shown in Table 1 relate to the first exemplary embodiment of the present invention. In Table 1, it may be seen that the catalyst may be removed with high efficiency by using a specific type of adsorbent, like the first exemplary embodiment of the present invention.

**[Table 2]**

| Classification | | Ex. 3 | Ex. 4 | Com. Ex. 5 | Com. Ex. 6 | Com. Ex. 7 | Com. Ex. 8 |
|---|---|---|---|---|---|---|---|
| Na (mg/L) | Raw material | 130.3 | 130.3 | 130.3 | 130.3 | 130.3 | 130.3 |
| | 1 µm | - | - | - | 77.2 | 77.2 | - |
| Adsorbent | Type | B | A | B | C1 | C2 | C2 |
| | Shape | Bead | Bead | Extrudat e | Bead | Bead | Bead |
| Na Content (mg/L) | After passing through adsorption tower | 141 | 20.7 | 2.41 | 44.7 | 29.4 | 687 |
| Presence or absence of breakage | | Absence | Absenc e | Presence | Presence | Presence | Presence |
| A: Si-Al | | | | | | | |
| B: Boehmite(Al) | | | | | | | |
| C1: Silica gel W127 | | | | | | | |
| C2: | Silica gel B127 | | | | | | |

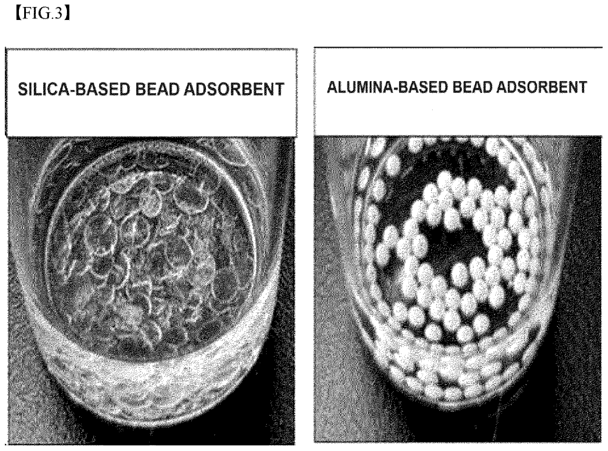

FIG. 3 is a photograph when adsorbents were added to reaction products in Comparative Example 7 (left) and Example 3 (right) of the present invention. Specifically, FIG. 3 is a photograph when a portion of the reaction product of each reaction was placed in a beaker with the adsorbents applied in Example 3 and Comparative Example 7 in the beaker, separately from adding the adsorbent to a quartz tube in each of Example 3 and Comparative Example 7. As may be seen in FIG. 3, the silica-based bead adsorbent of Comparative Example 7 was broken by an alcoholbased solvent in the reaction product. On the other hand, the alumina-based bead adsorbent of Example 3 was not broken by ethanol in the reaction product.

Examples 3 and 4 and Comparative Examples 5 to 8 shown in Table 2 relate to the second exemplary embodiment of the present invention. In Table 2, it may be seen that the catalyst may be removed with high efficiency without particle breakage by using an adsorption tower applied with a specific type of adsorbent, like the second exemplary embodiment of the present invention.

## Claims

1. A method for producing a carbonate comprising:
a reaction step of reacting raw materials containing dimethyl carbonate and ethanol in the presence of a catalyst to prepare a reaction product stream containing a catalyst, ethyl methyl carbonate, diethyl carbonate and methanol;
an adsorption step of adding an acidic adsorbent into the reaction product stream to induce adsorption of the catalyst and the adsorbent;
an adsorbent removal step of removing the adsorbent adsorbed with the catalyst to prepare a reaction product stream from which the adsorbent has been removed; and
a product separation step of separating ethyl methyl carbonate or diethyl carbonate from the reaction product stream from which the adsorbent has been removed,
wherein the adsorbent is an aluminum-based adsorbent.

2. A method for producing a carbonate comprising:
a reaction step of reacting raw materials containing dimethyl carbonate and ethanol in the presence of a catalyst to prepare a reaction product stream containing a catalyst, ethyl methyl carbonate, diethyl carbonate and methanol;
an adsorption step of supplying the reaction product stream to an adsorption tower including an acidic adsorbent to induce adsorption of the catalyst and the adsorbent; and
a product separation step of separating ethyl methyl carbonate or diethyl carbonate from the reaction product stream passing through the adsorption tower,
wherein the adsorbent is a bead-shaped aluminum-based adsorbent.

3. The method for producing the carbonate of claim 1 or 2, wherein the reaction step is performed in a continuous flow reactor.

4. The method for producing the carbonate of claim 1 or 2, wherein the catalyst includes sodium methoxide, sodium hydroxide, sodium ethoxide, potassium methoxide, potassium hydroxide, potassium ethoxide, or a combination thereof.

5. The method for producing the carbonate of claim 1 or 2, wherein the adsorbent is aluminum silicate or boehmite.

6. The method for producing the carbonate of claim 1 or 2, wherein in the product separation step, ethyl methyl carbonate or diethyl carbonate is separated by using a flash drum from the reaction product stream from which the adsorbent has been removed.

7. The method for producing the carbonate of claim 1, wherein in the adsorbent removal step, the catalyst and the adsorbed adsorbent are removed by filtration.

8. The method for producing the carbonate of claim 1, further comprising:
after the reaction step and before the adsorption step,
a preliminary catalyst removal step of filtering the reaction product stream.

9. The method for producing the carbonate of claim 2, wherein the product separation step is performed immediately after the adsorption step.
